# EUROPEAN PATENT APPLICATION

(11) **EP 4 679 097 A1**
(43) Date of publication of application: **14.01.2026**
(21) Application number: 24767036.7
(22) Date of filing: 01.03.2024
(51) Int. Cl.: G01N 33/68, G01N 27/62

(54) **METHOD FOR ESTIMATING BRAIN NEURODEGENERATIVE CONDITION**

(30) Priority: 03.03.2023 JP 2023032532
(71) Applicant: SHIMADZU CORPORATION, Kyoto-shi, Kyoto 604-8511 (JP); National Center for Geriatrics and Gerontology, Aichi 474-8511 (JP)
(72) Inventor: KANEKO, Naoki, Kyoto-shi, Kyoto 604-8511 (JP); NAKAMURA, Akinori, Obu-shi, Aichi 474-8511 (JP)
(74) Representative: Müller-Boré & Partner Patentanwälte PartG mbB
(86) International application number: PCT/JP2024/007692
(87) International publication number: WO 2024/185667

(57) **Abstract**

Provided is a method capable of easily and accurately estimating a neurodegenerative condition. A method for estimating a brain neurodegenerative condition includes: a measurement step of measuring a blood sample collected from a subject to obtain measured values of a plurality of neurogranin-related peptides; and a use step of calculating a specific value of a ratio using the measured values of the plurality of neurogranin-related peptides, and using the specific value of the ratio to determine a neurodegenerative condition.

## Description

### TECHNICAL FIELD

The present invention relates to a method for estimating a brain neurodegenerative condition.

### BACKGROUND ART

The number of dementia patients is estimated to be about 50 million worldwide in 2017, and is expected to be about 82 million in 2030. The number of dementia patients is estimated to reach 7 million also in Japan in 2025. The causes of dementia are classified into neurodegenerative disorder, vascular dementia, and other causes. Among them, the neurodegenerative disorder accounts for the largest proportion of the cause, and Alzheimer's disease (AD) accounts for more than half of all dementias. Non-Patent Documents 1 to 2 and Patent Documents 1 to 3 report that AB peptide can be used as a biomarker for determining the onset of Alzheimer's disease since amyloid B (AB) peptide generated by cleavage of amyloid precursor protein is deeply involved in the onset of Alzheimer's disease with respect to death of brain nerve cells.

Meanwhile, Neurogranin (Ng) is a protein which is distributed in large amounts in dendritic spines of brain nerve cells. Ng is a neuron-specific postsynaptic protein consisting of 78 amino acid residues, has a function of regulating calmodulin-dependent signaling, and is involved in synaptic plasticity. Non-Patent Documents 3 and 4 report that Ng is also one of the biomarkers of neurodegeneration and synaptic dysfunction, and the concentration of Ng increases in cerebrospinal fluid (CSF) of AD patients. Non-Patent Document 5 reports that in the brain of AD patients, fragmentation of Ng is promoted, and post-translationally modified Ng such as acetylation and glutathionylation is present. In addition, Non-Patent Document 5 reports that the ratio of the total value of the three types of post-translationally modified Ng1-78 to the Ng fragment peptide in CSF is increased in AD patients compared with the standard sample. Non-Patent Documents 6 and 7 report that Ng48-76 in CSF is increased in AD patients as compared with the standard sample. Thus, Ng has been reported as a marker reflecting neurodegeneration of AD patients in CSF.

### PRIOR ART DOCUMENTS

### PATENT DOCUMENTS

Patent Document 1: JP 6424757 B
Patent Document 2: JP 6410810 B
Patent Document 3: JP 6467512 B

### NON-PATENT DOCUMENTS

Non-Patent Document 1: Kaneko N, et al. : Novel plasma biomarker surrogating cerebral amyloid deposition. Proc Jpn Acad Ser B Phys Biol Sci. 2014; 90(9):353-364.
Non-Patent Document 2: Nakamura A, et al. : High performance plasma amyloid-B biomarkers for Alzheimer's disease. Nature. 2018; 554(7691):249-254.
Non-Patent Document 3: Portelius E, et al. : Cerebrospinal fluid neurogranin concentration in neurodegeneration: relation to clinical phenotypes and neuropathology. Acta Neuropathol. 2018; 136(3):363-376.
Non-Patent Document 4: Thorsell A, et al. : Neurogranin in cerebrospinal fluid as a marker of synaptic degeneration in Alzheimer's disease. Brain Res. 2010; 1362:13-22.
Non-Patent Document 5: Kvartsberg H, et al. : The intact postsynaptic protein neurogranin is reduced in brain tissue from patients with familial and sporadic Alzheimer's disease. Acta Neuropathol. 2019; 137(1):89-102.
Non-Patent Document 6: Kvartsberg H, et al. : Characterization of the postsynaptic protein neurogranin in paired cerebrospinal fluid and plasma samples from Alzheimer's disease patients and healthy controls. Alzheimers Res Ther. 2015 Jul 1; 7(1):40.
Non-Patent Document 7: Kvartsberg H, et al. : Cerebrospinal fluid levels of the synaptic protein neurogranin correlates with cognitive decline in prodromal Alzheimer's disease. Alzheimers Dement. 2015; 11(10):1180-90

### SUMMARY OF THE INVENTION

### PROBLEMS TO BE SOLVED BY THE INVENTION

By the way, the method of determining an AD patient using biomarkers in CSF involves extraction of CSF, and thus has high invasiveness to the patient. Therefore, analysis using a blood test that is less invasive and allows easy collection, and biomarkers thereof are required. However, analysis of Ng and its fragment peptide in blood has hardly progressed, and its effectiveness as a biomarker for dementia involving neurodegeneration has not been reported.

Then, the present inventors examined Ng and a fragment peptide thereof in a blood sample, and found that a specific Ng and a fragment peptide thereof are increased or decreased in patients with dementia caused by neurodegeneration, and thus are useful as biomarkers for the onset of dementia. As a result of further studies, the present inventors have found that the blood sample may be cryopreserved or stored at room temperature before analysis, and particularly after storage at room temperature, Ng and fragment peptides thereof are decomposed and the amount thereof varies. Therefore, the present inventors have found that the influence of the increase or decrease due to storage at room temperature is not considered only by measuring only the increase or decrease in a specific Ng or a fragment peptide thereof, and the increase or decrease in Ng or a fragment peptide thereof due to dementia caused by neurodegeneration may not be accurately measured, leading to lack of accuracy in determining the neurodegenerative condition.

An object of the present invention is to provide a method capable of easily and accurately estimating a neurodegenerative condition.

### MEANS FOR SOLVING THE PROBLEMS

A method for estimating a brain neurodegenerative condition according to a first aspect of the present invention relates to a method for estimating a brain neurodegenerative condition, the method including: a measurement step of measuring a blood sample collected from a subject to obtain measured values of a plurality of neurogranin-related peptides; and a use step of using at least one of the ratio values (1) to (11) described later from among the measured values of the plurality of neurogranin-related peptides to determine a neurodegenerative condition.

### EFFECTS OF THE INVENTION

According to the estimation method of the first aspect of the present invention, since the brain neurodegenerative condition of the patient can be easily and accurately estimated, the estimation method can be used for determining the presence or absence of the onset of dementia caused by neurodegeneration.

### BRIEF DESCRIPTION OF THE DRAWINGS

FIG. 1 shows a graph of a variation rate between the normalized intensity (NI) of a specimen which is thawed, placed at room temperature for about 3 hours, and then re-frozen (specimen under RT3h-FT1 conditions) and the normalized intensity of a specimen not subjected to the process (specimen under control conditions).

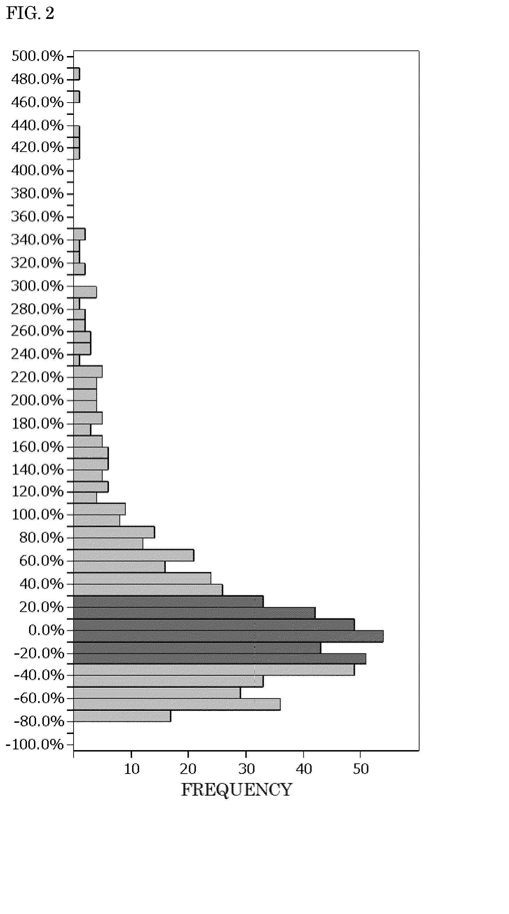

FIG. 2 shows a distribution diagram of variation rates in all Ng ratios in the graph of FIG. 1.
FIG. 3 shows a correlation plot diagram between a Ng ratio ((1) to (4)) showing a high value in dementia and an FDG-PET score. The vertical axis represents NI of each Ng peptide, and the horizontal axis represents FDG-PET score.
FIG. 4 shows a correlation plot diagram between a Ng ratio ((5) to (8)) showing a high value in dementia and an FDG-PET score.
FIG. 5 shows a correlation plot diagram between a Ng ratio ((9) to (11)) showing a high value in dementia and an FDG-PET score.
FIG. 6 shows a correlation plot diagram between a Ng ratio ((1) to (4)) showing a high value in dementia and VSRAD. The vertical axis represents NI of each Ng peptide, and the horizontal axis represents VSRAD.
FIG. 7 shows a correlation plot diagram between a Ng ratio ((5) to (8)) showing a high value in dementia and VSRAD.
FIG. 8 shows a correlation plot diagram between a Ng ratio ((9) to (11)) showing a high value in dementia and VSRAD.
FIG. 9 shows a correlation plot diagram between a Ng ratio ((1) to (4)) showing a high value in dementia and MMSE. The vertical axis represents NI of each Ng peptide, and the horizontal axis represents MMSE.
FIG. 10 shows a correlation plot diagram between a Ng ratio ((5) to (8)) showing a high value in dementia and MMSE.
FIG. 11 shows a correlation plot diagram between a Ng ratio ((9) to (11)) showing a high value in dementia and MMSE.

### MODE FOR CARRYING OUT THE INVENTION

### 1. First embodiment

An estimation method according to a first embodiment is a method for determining a brain neurodegenerative condition, and includes a measurement step and a use step in order.

### 1-1. Measurement step

In the measurement step, a blood sample is measured to analyze neurogranin-related peptides in the blood sample. As an example of a preferred measurement step of the first embodiment, the blood sample is sequentially subjected to purification treatment and mass spectrometry. That is, a blood sample containing blood collected from a subject is purified, and then subjected to mass spectrometry to obtain measured values of neurogranin-related peptides in the blood sample. Hereinafter, this embodiment will be described in detail.

### (Purification treatment)

The purification treatment of the blood sample is preferably, for example, affinity purification. Impurities contained in the blood sample can be removed through the purification, and a trace amount of the neurogranin-related peptide contained in the blood can be reliably detected.

In the first embodiment, the "neurogranin-related peptide" (hereinafter, abbreviated as "Ng peptide") includes, in addition to neurogranin (Ng1-78) (SEQ ID NO: 36) and a fragment peptide thereof, a modified neurogranin such as acetylated neurogranin or cysteinylated neurogranin or a fragment peptide thereof. Examples of the Ng peptide include the peptides listed in Tables 3 and 4 in the Examples described below.

The affinity purification may be performed once or a plurality of times, but the purification is preferably performed twice from the viewpoint of being able to detect the Ng peptide with higher sensitivity. In this case, the purification step includes a first binding step, a first washing step, a first elution step, a neutralization step, a second binding step, a second washing step, and a second elution step in this order.

In the first binding step, the blood sample is brought into contact with a first carrier. As a result, the Ng peptide in the blood sample is bound to the first carrier to obtain a first conjugate.

The blood sample is a sample solution containing blood collected from a subject, and is preferably, for example, blood itself, or a diluted solution thereof. The blood sample includes whole blood, plasma, serum, and the like. The blood sample may be obtained by subjecting whole blood collected from an individual to a treatment such as centrifugation and cryopreservation.

Examples of the diluted solution of the blood sample include a mixed solution of blood and a buffer. Examples of the buffer include a Tris buffer, a phosphate buffer, a HEPES buffer, and an ammonium acetate buffer.

The diluted solution may be mixed with, for example, an organic solvent such as dimethylformamide or acetonitrile. The final concentration of the organic solvent is, for example, 5 to 30 (v/v)%. As a result, it is possible to suppress decomposition of a desired Ng peptide over time during the storage of the blood sample collected from a subject.

The diluted solution may also be mixed with a surfactant. Examples of the surfactant include neutral surfactants having a hydrophobic group having 7 or more and 15 or less carbon atoms. The concentration of the surfactant is, for example, 0.01 to 5 (v/v)%. As a result, it is possible to suppress non-specific adsorption to the first conjugate and to reduce ionization interference in mass spectrometry. Examples of such a surfactant include surfactants having maltose in a hydrophilic moiety, such as n-nonyl-β-D-maltoside, n-nonyl-β-D-thiomaltoside, n-decyl-β-D-maltoside, and n-undecyl-β-D-maltoside (UDM: n-Undecyl-β-D-maltoside); surfactants having trehalose in a hydrophilic moiety, such as α-D-glucopyranosyl-α-D-glucopyranoside monodecanoate (trehalose C10); and surfactants having glucose in a hydrophilic moiety, such as n-decyl-β-D-glucoside.

The blood sample is preferably further mixed with an internal standard in order to normalize or standardize the measured value. The internal standard preferably includes a peptide labeled with a stable isotope. The amount of the internal standard to be mixed is, for example, 1 pM or more and 1 nM or less with respect to the amount of the plasma sample.

The first carrier may be any carrier to which the Ng peptide can be bound, and examples thereof include antibody-immobilizing carriers. The antibody immobilized on the first carrier is an antibody having antigen binding sites capable of recognizing the Ng peptide (anti-Ng peptide antibody). Examples of the antibody include an immunoglobulin having antigen binding sites capable of recognizing the Ng peptide or a fragment thereof. Examples of the immunoglobulin include IgG (IgG1, IgG2, IgG3, and IgG4), IgM, IgA, IgY, IgD, and IgE. Examples of the immunoglobulin fragment include F(ab')2, F(ab'), F(ab), Fd, Fv, L chain, and H chain. More specifically, the immunoglobulin fragment is clones NG2, NG7, EPR21152, fragments thereof, and the like.

In the first washing step, after the first binding step, the first conjugate is washed with a first washing solution.

The first washing solution is preferably a neutral buffer containing the surfactant described above. This makes it possible to effectively remove highly hydrophobic undesirable components (for example, blood proteins, lipids, and glycolipids).

In the washing method, washing is preferably performed a plurality of times. For example, washing is performed with a neutral buffer containing a surfactant, followed by washing with a neutral buffer containing no surfactant. As the washing method, a general method may be employed, and examples thereof include a method of stirring a carrier in a washing solution, and a method of spraying a washing solution from a washing nozzle. After washing with these neutral buffers, washing with water may be further performed as necessary.

In the first elution step, after the first washing step, the first conjugate is brought into contact with a first acidic solution. As a result, the Ng peptide is dissociated from the first conjugate, and the Ng peptide is eluted into the first acidic solution. As a result, a first eluate containing the Ng peptide is obtained.

Examples of the first acidic solution include acidic aqueous solutions (pH 0.5 to 3.5) such as a glycine buffer and hydrochloric acid. The first acidic solution preferably contains the surfactant described above. As a result, the Ng peptide can be more reliably dissociated from the first conjugate, and thus the detection sensitivity can be improved.

In the neutralization step, after the first elution step, the first eluate is mixed with a neutral buffer. As a result, the first eluate is neutralized to obtain a purified solution containing the Ng peptide.

The neutral buffer used in the neutralization step preferably contains the surfactant described above. This makes it possible to suppress non-specific adsorption to the second conjugate in the second binding step.

The pH of the resulting purified solution is neutral, for example, pH 6.0 or more, preferably 6.5 or more, and is, for example, 8.5 or less, preferably 8.0 or less. Accordingly, the binding efficiency can be improved in the second binding step.

In the second binding step, after the neutralization step, the purified solution is brought into contact with a second carrier. As a result, the Ng peptide of the purified solution is bound to the second carrier to obtain a second conjugate.

The second carrier is preferably an antibody-immobilizing carrier, and specific examples thereof include the same antibody-immobilizing carriers as exemplified for the first carrier.

In the second washing step, after the second binding step, the second conjugate is washed with a second washing solution.

The second washing solution is preferably a neutral buffer containing the surfactant described above. As a result, for example, unnecessary components having high hydrophobicity can be effectively removed.

As the washing method, a known method may be employed, and specifically, a method similar to the washing method exemplified in the first washing step may be performed.

In the second elution step, after the second washing step, the second conjugate is brought into contact with a second acidic solution. As a result, the Ng peptide is dissociated from the second conjugate, and eluted into the second acidic solution. As a result, a second eluate containing the Ng peptide is obtained.

Examples of the acidic aqueous solution constituting the second acidic solution include those similar to the first acidic solution exemplified in the first elution step, and preferably include hydrochloric acid.

The second acidic solution preferably contains a volatile organic solvent. As a result, the Ng peptide can be efficiently dissociated from the second conjugate and eluted into the second acidic solution, so that the recovery rate of the Ng peptide can be improved. Examples of the volatile organic solvent include organic solvents miscible with water in an arbitrary ratio. Examples thereof include acetonitrile, methanol, ethanol, acetone, toluene, isopropanol, hexane, butanol, cyclohexane, ethylene glycol, benzene, chloroform, acetaldehyde, triethylamine, phenol, naphthalene, formaldehyde, tetrahydrofuran, and ethyl acetate.

The second acidic solution preferably further contains an amino acid such as methionine. As a result, the oxidation of the Ng peptide can be reduced before the sample is placed in the mass spectrometer and analysis is started, and thus the detection sensitivity can be improved.

The second acidic solution preferably further contains a protein of 9 kDa or more. Since the protein is contained in the mass spectrometry sample, the protein acts as a proton receptor during mass spectrometry, and allows monovalent ions of the Ng peptide to be efficiently generated. As a result, the detection sensitivity of the Ng peptide can be remarkably improved. The upper limit of the mass of the protein is, for example, 100 kDa or less, preferably 15 kDa or less. Specific examples of such a protein include bovine serum albumin (BSA), cytochrome, ovalbumin, and lysozyme.

### (Mass spectrometry)

After the purification treatment, the second eluate is subjected to mass spectrometry to thereby detect the Ng peptide.

Examples of the ionization method in mass spectrometry include Matrix Assisted Laser Desorption/Ionization (MALDI); Electrospray Ionization (ESI); and Atmospheric Pressure Chemical Ionization (APCI). MALDI is preferable from the viewpoint that a trace amount of Ng peptide in the plasma sample can be detected with high sensitivity.

Examples of the mass spectrometry include, but are not limited to, time-of-flight mass spectrometry (TOF-MS), ion trap mass spectrometry (IT-MS), ion trap-time-of-flight mass spectrometry (IT-TOF-MS), and Fourier transform ion cyclotron mass spectrometry (FTICR-MS). When MALDI is used as the ionization method, the mass spectrometry is collectively referred to as MALDI-MS.

As a specific detection operation by MALDI-MS, for example, first, a matrix-containing solution is added dropwise to a MALDI plate and dried to arrange the matrix. Subsequently, the second eluate is added dropwise to the matrix and dried to obtain a MALDI-MS sample. Subsequently, the MALDI-MS sample is irradiated with a laser to ionize the Ng peptide, and the ionized peptide is detected by a detector of the above apparatus and output as a mass spectrum.

Examples of the matrix include α-cyano-4-hydroxycinnamic acid, 2,5-dihydroxybenzoic acid, sinapinic acid, and 3-aminoquinoline. A matrix additive is preferably used in combination with the matrix. Examples of the matrix additive include phosphonic acid group-containing compounds such as methylene diphosphonic acid; and ammonium salts. Examples of the solvent in which the matrix is allowed to be contained include acetonitrile, trifluoroacetic acid, methanol, ethanol, and water.

As a result, a measured value showing the peak intensity (including the peak area) of the ion peak corresponding to each of the plurality of Ng peptides is obtained together with mass spectra of a plurality of detected Ng peptides contained in the blood sample. Examples of such a Ng peptide include those in Examples described later (see Tables 3 and 4). This measured value may be a relative intensity with the intensity of the reference peak taken as 100%, or may be an intensity normalized by an internal standard (NI: normalized Intensity). When the second eluate is divided into a plurality of portions (plurality of wells) and measured to obtain a plurality of measured values (including normalized intensities), the average value thereof may be used.

In addition, the Ng peptide detected by mass spectrometry may be detected as a peak of a monovalent ion, or may be detected at peaks of a monovalent ion and a multivalent ion such as a divalent or higher ion. For example, both ions are detected in the case of Ng43-75 and the like. In this case, any peak intensity may be used as the measured value of the biomarker, but in the case of MALDI-MS, preferably, the peak intensity detected at the peak of the monovalent ion is used for small Ng peptides having a mass of less than 5000, and the peak intensity detected at the peak of the divalent ion is used for large Ng peptides having a mass of 5000 or more. On the other hand, in the case of Electrospray Ionization (ESI), the peak intensity detected at the peak of the multivalent ion is preferably used.

### 1-2. Use step

In the use step, a specific ratio is calculated using the measured values of the plurality of Ng peptides obtained in the measurement step, and is used to determine the neurodegenerative condition. That is, the measured values of the plurality of specific Ng peptides are selected from among many measured values of Ng peptides detected in the mass spectrum obtained in the measurement step. Then, the ratio of these measured values is calculated and used as an index, a score, or the like for determining the neurodegenerative condition.

In the first embodiment, the expression "used to determine a neurodegenerative condition" means that a specific Ng peptide ratio (including values such as normalized values and average values) (the Ng ratio) serving as a biomarker may be used as part of the basis for determining the neurodegenerative condition. For example, use of the Ng peptide ratio includes, in addition to (1) the case where the Ng ratio is used as an index for determination as it is, (2) the case where the Ng ratio is corrected or processed into a variable, and the corrected value or the variable is used as an index for determination, (3) the case where the Ng ratio is made reciprocal and the value is used as an index for determination, (4) the case where a desired value or an equation is added to, subtracted from, multiplied by or divided by the Ng ratio, and the added, subtracted, multiplied or divided value is used as an index for determination, and (5) a combination thereof.

The Ng ratio serving as a biomarker is at least one ratio of the following (1) to (11). The measured value of Ng52-75 is a measured value detected for an Ng peptide of Ng52-75 when a blood sample is measured, and includes the concentration and amount of Ng52-75 or a numerical value similar thereto. In mass spectrometry, as described above, the measured value is the peak intensity (including normalized intensity, average value, and the like). The sequences of Ng peptides that are subjects of these ratios are shown in Table 1 below:
(1) the ratio of the measured value of Ng53-75 to the measured value of Ng52-75;
(2) the ratio of the measured value of Ng53-78 to the measured value of Ng51-75;
(3) the ratio of the measured value of Ng43-77 to the measured value of Ng43-75;
(4) the ratio of the measured value of Ng43-77 to the measured value of AcNg1-78ss;
(5) the ratio of the measured value of Ng43-77 to the sum of measured values of AcNg1-75ss and AcNg1-78ss;
(6) the ratio of the measured value of Ng43-77 to the sum of measured values of AcCysNg1-75ss and AcCysNg1-78ss;
(7) the ratio of the measured value of Ng43-77 to the sum of measured values of Ng24-75 and Ng24-78;
(8) the ratio of the measured value of Ng43-78 to the measured value of Ng51-75;
(9) the ratio of the measured value of Ng24-78 to the measured value of AcCysNg1-75ss;
(10) the ratio of the sum of measured values of Ng53-75 and Ng53-78 to the measured value of Ng52-75; and
(11) the ratio of the sum of measured values of Ng53-75 and Ng53-78 to the measured value of Ng51-75.

**[Table 1]**

| Ng peptide | Sequence | No. |
|---|---|---|
| Ng53-75 | RKGPGPGG PGGAGVARGG AGGGP | 1 |
| Ng53-78 | RKGPGPGG PGGAGVARGG AGGGPSGD | 2 |
| Ng43-77 | RKKIKSGE RGRKGPGPGG PGGAGVARGG AGGGPSG | 14 |
| Ng43-78 | RKKIKSGE RGRKGPGPGG PGGAGVARGG AGGGPSGD | 5 |
| Ng24-75 | PGANAAA AKIQASFRGH MARKKIKSGE RGRKGPGPGG PGGAGVARGG AGGGP | 24 |
| Ng24-78 | PGANAAA AKIQASFRGH MARKKIKSGE RGRKGPGPGG PGGAGVARGG AGGGPSGD | 25 |
| Ng52-75 | GRKGPGPGG PGGAGVARGG AGGGP | 6 |
| Ng44-75 | KKIKSGE RGRKGPGPGG PGGAGVARGG AGGGP | 4 |
| Ng51-75 | RGRKGPGPGG PGGAGVARGG AGGGP | 7 |
| Ng43-75 | RKKIKSGE RGRKGPGPGG PGGAGVARGG AGGGP | 13 |
| AcNg1-75ss | MDCCTENACS KPDDDILDIP LDDPGANAAA AKIQASFRGH MARKKIKSGE RGRKGPGPGG PGGAGVARGG AGGGP | 28 |
| AcNg1-78ss | MDCCTENACS KPDDDILDIP LDDPGANAAA AKIQASFRGH MARKKIKSGE RGRKGPGPGG PGGAGVARGG AGGGPSGD | 30 |
| AcCysNg1-75ss | MDCCTENACS KPDDDILDIP LDDPGANAAA AKIQASFRGH MARKKIKSGE RGRKGPGPGG PGGAGVARGG AGGGP | 29 |
| AcCysNg1-78ss | MDCCTENACS KPDDDILDIP LDDPGANAAA AKIQASFRGH MARKKIKSGE RGRKGPGPGG PGGAGVARGG AGGGPSGD | 31 |

Abbreviations in the table are as follows. Ac: Acetylation, Cys: Cysteinylation, Glu: Glutathionylation, ss: internal disulfide bond Acetylation occurs at the first amino acid of Ng1-78, glutathionylation occurs at the third or ninth amino acid, and an internal disulfide bond occurs between the third and fourth amino acids or between the fourth and ninth amino acids (see Non-Patent Document 5).

These biomarkers may be used alone or in combination. The value of at least one of (1) to (2), the value of at least one of (3) to (7), or the value of at least one of (10) to (11) is preferable from the viewpoint of having high analysis precision for both Alzheimer's disease and non-Alzheimer's dementia.

The value of the Ng ratio to be a biomarker is then used to determine the neurodegenerative condition.

Specifically, the Ng ratio of the biomarker in a blood sample collected from a subject is compared with a predetermined value. Then, when the Ng ratio of the biomarker is higher or lower than a predetermined value, it is determined that neurodegeneration has occurred in the brain of the subject and dementia has developed in the subject. Examples of the aspect of comparison include 1) an aspect in which a Ng ratio in a blood sample collected from a subject is compared with a preset reference value of normal cognitive function; 2) an aspect in which Ng ratios of a plurality of blood samples collected over time (for example, every few months) from a subject are collected and the Ng ratios of the plurality of blood samples are compared; and 3) a combination thereof. The reference value of 1) can be appropriately set from the Ng ratios of blood samples collected from a plurality of persons with normal cognitive function and/or dementia in advance.

In particular, the Ng ratios of the above (1) to (11) increase in the blood of a subject in whom neurodegeneration has occurred. Therefore, in the aspect of 1), when the Ng ratio in the subject is higher than the reference value of normal cognitive function, it is determined that neurodegeneration has occurred in the brain of the subject. In the aspect of 2), when the Ng ratio measured on a specific day is higher than the Ng ratio measured on a previous day, it is determined that neurodegeneration has occurred or has progressed in the brain of the subject. On the other hand, in the opposite case, that is, in the aspects 1) and 2), when the former measured value is low, it is determined that the brain neurodegeneration of the subject is suppressed.

As described above, the estimation method using the value of Ng ratio as a biomarker according to the first embodiment is a method for detecting Ng peptides from a blood sample and using the detected Ng peptides. By measuring a blood sample collected from a subject, selecting measured values of the plurality of Ng peptides from measurement results, and calculating the ratios of the measured values and using the ratios, it is possible to easily estimate the brain neurodegenerative condition, that is, whether or not the functional loss of brain nerve cells has occurred. The estimation method enables, for example, easy prediction of the presence or absence and progress of a loss of synaptic function, synaptic loss, and the like. Therefore, in this method, the value of Ng ratio can be used as information for diagnosing whether or not the subject has developed dementia caused by neurodegeneration. Specifically, the value of Ng ratio can be used as information for diagnosing dementia including non-Alzheimer's dementia (Lewy body dementia, frontotemporal dementia, and the like), in addition to Alzheimer's disease which is representative as dementia caused by neurodegeneration. Furthermore, neurodegeneration appears earlier than the onset of dementia due to a mechanism that the onset of dementia is caused by neurodegeneration. Therefore, the value of Ng ratio can also be used as information for predicting the onset of these dementias or diagnosing the onset risk of dementias.

In particular, this estimation method enables easy estimation of the brain neurodegenerative condition even for a blood sample after thawing and storage. When a blood sample is collected from an individual or treated by centrifugation, the blood sample is in a liquid state and is present at room temperature or a low temperature of 0°C or higher. The blood sample is generally cryopreserved to keep the freshness. The time from blood collection to cryopreservation varies depending on the medical institution and the blood collection site. The blood sample includes a blood sample obtained by thawing the cryopreserved blood sample into a liquid state, then storing the blood sample at normal temperature or a low temperature of 0°C or higher for several hours, and then re-freezing the blood sample. In such a blood sample, the amount of the Ng peptide increases or decreases in a liquid state before cryopreservation, and the Ng peptide at the time of blood collection varies. The reason for the variation is considered to be that in the liquid state, enzymes in the blood act to decompose the Ng peptide and generate decomposition products at the same time. The state at the time of blood collection in such a case may not be accurately determined. On the other hand, in this estimation method, the biomarker is set to a specific Ng peptide ratio, that is, a specific Ng peptide measurement amount that varies is set to a relative value with the specific other Ng peptide measurement amount that varies. Thereby, it is possible to reduce the influence of the variation of the Ng peptide in the liquid state, and thus accurately estimate the brain neurodegenerative condition.

This estimation method can also assess the effectiveness of the medical intervention. For example, if it is determined that the neurodegeneration has occurred in the subject, the implementation of the medical intervention is started, and then the estimation method is performed again to determine the state of the neurodegeneration. In performing the estimation method again, if it is determined that the progress of neurodegeneration is stopped, suppressed, delayed, or there is no neurodegeneration, the medical intervention can be evaluated to be effective. If it is determined that the neurodegeneration is progressing, the medical intervention can be evaluated to be ineffective. Furthermore, the estimation method can also be applied to a case where it is determined that neurodegeneration has occurred, and thereafter, whether or not the neurodegeneration has progressed is followed up even in a state where there is no medical intervention.

### 1-3. Modified Example

1). In the above embodiment, the Ng ratios of (1) to (11) are adopted and used for determination of the neurodegenerative condition, but for example, a variable is calculated by multivariate analysis using a combination of the plurality of measured values, and this variable may be used for determination of the neurodegenerative condition. That is, measured values including two selected from the group consisting of (1) to (11) are selected, and these selected measured values are calculated as a variable by multivariate analysis. This variable may be used for determination of the neurodegenerative condition. As a result, an area under curve (AUC) in ROC analysis is further improved, and more accurate determination can be made.

As the multivariate analysis, a known mathematical method can be used. Examples of the multivariate analysis include discriminant analysis (linear discriminant analysis, quadratic discriminant analysis, normalized discriminant analysis, and the like), multiple regression analysis, kernel method, principal component analysis, partial least squares regression, logistic regression, and Z-score.

2). In the above embodiment, the mass spectrometry is performed as the measurement step, but the measurement method is not limited, and may be, for example, a sandwich ELISA. In this method, the Ng peptide to be a biomarker can be measured by sandwiching with an N-terminal-specific antibody for each of the Ng peptide and an antibody that recognizes the C-terminal region of the Ng peptide. In addition, the Ng peptide including Ng53 as the N-terminal in the blood sample is mainly composed of Ng53-75 and Ng53-78. The other Ng peptides including Ng53 as the N-terminal are present so minute that they cannot be detected by mass spectrometry, and thus hardly affect the combined value of Ng53-75 and Ng53-78. Therefore, the combined value of Ng53-75 and Ng53-78 can be measured by sandwiching with an N-terminal-specific antibody for Ng53-75 and an antibody that recognizes the C-terminal region from N53.

Note that the measured value of each Ng peptide is appropriately determined according to the measurement method, but may be, for example, a concentration, an absorbance, or the like.

### 2. Aspects

It is understood by those skilled in the art that the plurality of exemplary embodiments described above are specific examples of the following aspects.

(Item 1) A method for estimating a brain neurodegenerative condition according to one aspect may include: a measurement step of measuring a blood sample collected from a subject to obtain measured values of a plurality of neurogranin-related peptides; and a use step of calculating at least one of the values of the ratios (1) to (11) using the measured values of the plurality of neurogranin-related peptides, and using the at least one of the values of the ratios to determine a neurodegenerative condition:
(1) a ratio of a measured value of Ng53-75 to a measured value of Ng52-75;
(2) a ratio of a measured value of Ng53-78 to a measured value of Ng51-75;
(3) a ratio of a measured value of Ng43-77 to a measured value of Ng43-75;
(4) a ratio of a measured value of Ng43-77 to a measured value of AcNg1-78ss;
(5) a ratio of a measured value of Ng43-77 to a sum of measured values of AcNg1-75ss and AcNg1-78ss;
(6) a ratio of a measured value of Ng43-77 to a sum of measured values of AcCysNg1-75ss and AcCysNg1-78ss;
(7) a ratio of a measured value of Ng43-77 to a sum of measured values of Ng24-75 and Ng24-78;
(8) a ratio of a measured value of Ng43-78 to a measured value of Ng51-75;
(9) a ratio of a measured value of Ng24-78 to a measured value of AcCysNg1-75ss;
(10) a ratio of a sum of measured values of Ng53-75 and Ng53-78 to a measured value of Ng52-75; and
(11) a ratio of a sum of measured values of Ng53-75 and Ng53-78 to a measured value of Ng51-75.

(Item 2) In the estimation method according to item 1, in the use step, when the value of the ratio is higher than a predetermined value, it may be determined that neurodegeneration has occurred in the brain of the subject.

(Item 3) In the estimation method according to item 1, in the use step, the value of the ratio may be compared with a preset reference value of normal cognitive function.

(Item 4) In the estimation method according to item 1, the measurement step may be performed a plurality of times over time, and the use step may be a step of comparing a plurality of values of ratios obtained and calculated by performing the step a plurality of times.

(Item 5) In the estimation method according to any one of items 1 to 4, in the use step, two types of values of ratios are selected from among ratios obtained by using the measured values of the plurality of neurogranin-related peptides, a variable is calculated using multivariate analysis, and the variable may be used to determine a neurodegenerative condition.

(Item 6) In the estimation method according to any one of items 1 to 5, in the use step, at least one of the values of the ratios (1) to (2) may be used.

(Item 7) In the estimation method according to any one of items 1 to 5, in the use step, at least one of the values of the ratios (3) to (7) may be used.

(Item 8) In the estimation method according to any one of items 1 to 5, in the use step, at least one of the values of the ratios (10) to (11) may be used.

(Item 9) In the estimation method according to any one of items 1 to 8, in the measurement step, mass spectrometry may be performed on the blood sample to obtain measured values of peak intensities for the plurality of neurogranin-related peptides.

(Item 10) In the estimation method according to item 9, affinity purification may be performed on the blood sample before mass spectrometry.

(Item 11) A method for detecting a neurogranin-related peptide, the method including:
a step of detecting at least one combination of neurogranin-related peptides selected from the group consisting of the following (1) to (11) in a blood sample:
(1) a combination of Ng52-75 and Ng53-75;
(2) a combination of Ng51-75 and Ng53-78;
(3) a combination of Ng43-75 and Ng43-77;
(4) a combination of AcNg1-78ss and Ng43-77;
(5) a combination of AcNg1-75ss, AcNg1-78ss, and Ng43-77;
(6) a combination of AcCysNg1-75ss, AcCysNg1-78ss, and Ng43-77;
(7) a combination of Ng24-75, Ng24-78 and Ng43-77;
(8) a combination of Ng51-75 and Ng43-78;
(9) a combination of AcCysNg1-75ss and Ng24-78;
(10) a combination of Ng52-75, Ng53-75, and Ng53-78; and
(11) a combination of Ng51-75, Ng53-75, and Ng53-78.

### EXAMPLES

Next, the present invention will be described in detail with reference to Examples, but the scope of the present invention is not limited thereto.

### 1. Plasma specimen

Plasma specimens (161 cases) and clinical diagnostic information such as amyloid PET findings, FDG-PET findings, MRI findings, and MMSE findings (mini mental state examination) were acquired at the National Center for Geriatrics and Gerontology. Detailed information is shown in Table 2.

**[Table 2]**

| Clinical diagnosis | | N | MMSE | FDG_PET score | VSRAD | Amyloid PET (PIB tracer) | | | |
|---|---|---|---|---|---|---|---|---|---|
| | | | Average value | Average value | Average value | SUVR Average value | Aβ negative (N) | Aβ positive (N) | Others (N) |
| CN (normal cognitive function) | | 62 | 28.9 | 0.53 | 0.75 | 1.24 | 49 | 13 | - |
| MCI (mild cognitive impairment) | | 13 | 27.5 | 0.71 | 1.28 | 1.59 | 6 | 7 | - |
| Dementia | AD | 40 | 20.2 | 1.71 | 1.50 | 1.89 | 1 | 39 | - |
| | nonAD | 46 | 22.6 | 1.44 | 1.49 | 1.10 | 41 | 3 | Undeterminable 1 Not performed 1 |

The subject is a case diagnosed with normal cognitive function (CN), mild cognitive impairment (MCI), Alzheimer's disease (AD), and dementia other than Alzheimer's disease (nonAD). A group of AD and nonAD was defined as a group of dementia.

An FDG-PET score quantifying a ¹⁸F-FDG-PET scan was used as FDG-PET findings (see Herholz K, et al.; Evaluation of a calibrated (18) F-FDG PET score as a biomarker for progression in Alzheimer's disease and mild cognitive impairment. J Nucl Med. 2011 Aug; 52 (8): 1218 to 26).

An index for atrophy assessment in the vicinity of the hippocampus by voxel-based morphometry using VSRAD was used as MRI findings (see Sone D, et al.; Japanese-Alzheimer's Disease Neuroimaging Initiative.Voxel-based Specific Regional Analysis System for Alzheimer's Disease (VSRAD) on 3-tesla Normal Database: Diagnostic Accuracy in Two Independent Cohorts with Early Alzheimer's Disease. AgiNg Dis. 2018 Aug 1; 9(4): 755 to 760).

PiB-PET images of the brains of the subjects were acquired in order to determine the positivity or negativity of cerebral AB accumulation. When the PiB accumulation amount in the cerebral cortex was larger than or equal to the non-specific PiB accumulation amount in the white matter in visual interpretation evaluation by a nuclear medicine specialist, the subject was determined to be positive. When only non-specific accumulation in the white matter was observed and little accumulation was observed in the cortex, the subject was determined to be negative. The PiB accumulation average value (SUVR: Standard Uptake Value Ratio) was obtained by quantifying the PiB accumulation in the cortex, and determining the accumulation ratio of the cerebrum to the cerebellum as a reference. AD cases confirmed to be AB-positive in amyloid PET were defined as AD_Aβ+ (N = 39), and nonAD cases confirmed to be AB-negative in amyloid PET were defined as nonAD_Aβ-(N = 41).

### <Experimental Example 1>

### 1. Measurement of Ng peptide in control sample

### [Preparation of blood sample]

Dimethyl sulfoxide (DMSO) was mixed with a first buffer (0.1% n-undecyl-β-D-maltoside (UDM), 800 mM GlcNAc, 100 mM Tris-HCl, 300 mM NaCl; pH 7.4) containing a surfactant to prepare a first buffer with a DMSO concentration of 20 (v/v)%. Subsequently, as an internal standard, Ng53-75 (SIL-Ng53-75), Ng45-75 (SIL-Ng45-75), and Ng24-75 (SIL-Ng24-75) labeled with a stable isotope were mixed with the first buffer so as to be 30 pM, 50 pM, and 50 pM, respectively, to prepare an internal standard-containing buffer. Subsequently, 200 µL of human plasma (control sample) was mixed with 200 µL of the internal standard-containing buffer, and the mixture was allowed to stand on ice for 5 to 60 minutes. Thus, a plasma sample was prepared. In SIL-Ng45-75 and SIL-Ng24-75, the carbon atoms and nitrogen atoms of some amino acids of Pro, Val, and Ala are substituted with ¹³C and ¹⁵N, respectively.

### [Purification]

### (First binding step, first washing step, and first elution step)

A clone NG2 (manufactured by BioLegend, Inc.) of an anti-Ng antibody (IgG1) having 52 to 63 residues of human Neurogranin (Ng) as an epitope was prepared. To 100 µg of the anti-Ng antibody, 5.5 mg of magnetic beads (Dynabeads M-270 Epoxy) were added in an immobilization buffer (0.1 M phosphate buffer containing 1.5 M ammonium sulfate; pH 7.4) at 37°C for 16 to 24 hours. Thus, antibody beads as an antibody-immobilizing carrier were prepared.

The plasma sample was mixed with the antibody beads, and the mixture was incubated at 4°C for 1 hour. The antibody beads were then washed once with 100 µL of a first washing buffer (0.05%UDM, 50 mM Tris-HCl, 150 mM NaCl; pH 7.4) and once with 50 µL of a 50 mM ammonium acetate buffer. Thereafter, the antibody beads were brought into contact with a first acidic solution (0.05%UDM, 50 mM glycine buffer; pH 2.8) to elute Ng peptides into the first acidic solution. Thus, a first eluate containing Ng peptides was obtained.

### (Neutralization step)

The first eluate was mixed with a neutral buffer (0.1%UDM, 800 mM GlcNAc, 300 mM Tris-HCl, 300 mM NaCl; pH 7.4) to obtain a purified solution.

### (Second binding step, second washing step, and second elution step)

The purified solution was mixed with antibody beads, and the mixture was incubated at 4°C for 1 hour. Thereafter, the antibody beads were washed twice with 50 µL of a second washing buffer (0.05%UDM, 50 mM Tris-HCl, 150 mM NaCl; pH 7.4), washed once with 50 µL of a 50 mM ammonium acetate buffer, and washed once with 30 µL of water. Thereafter, the antibody beads were brought into contact with a second acidic solution (70% (v/v) acetonitrile aqueous solution containing 5 mM hydrochloric acid and 0.1 mM methionine, 50 nM BSA) to elute Ng peptides into the second acidic solution. A second eluate containing Ng peptides was thus obtained.

### [Mass spectrometry]

AXIMA Performance (Shimadzu/KRATOS, Manchester, UK), which is a MALDI-TOF MS apparatus, was used as a mass spectrometer. A 2 mg/mL CHCA/0.2% (w/v) MDPNA matrix solution was prepared using α-cyano-4-hydroxycinnamic acid (CHCA) as a matrix for Linear TOF, methylene diphosphonic acid (MDPNA) as a matrix additive, and acetonitrile as a solvent. To four wells of a MALDI plate (µFocus MALDI plate 900 µm (Hudson Surface Technology, Inc., Fort Lee, NJ)), 0.5 µL each of the matrix solution was added dropwise and dried, and then 1 µL each of the second eluate was further added dropwise thereto and dried.

MALDI-TOF MS was then activated to detect Ng peptides. As a setting condition, mass spectrum data was acquired by Linear TOF in a positive ion mode. 400 spots and 16000 shots were accumulated for one well. The m/z value of Linear TOF was indicated by average mass of peaks. The m/z value was calibrated with human angiotensin II and human ACTH fragment 18-39, bovine insulin oxidized beta-chain, bovine insulin, and cytochrome c as external standards.

Tables 3 and 4 show the Ng peptides detected in the mass spectrum. The symbol * in the table represents the peak of the divalent ion.

**[Table 3]**

| Peptide name | Sequence | T heoret i cal average m/ z | No. |
|---|---|---|---|
| N g43-75* | RKKIKSGE RGRKGPGPGG PGGAGVARGG AGGGP | 1493.2 | 13 |
| N g43-78* | RKKIKSGE RGRKGPGPGG PGGAGVARGG AGGGPSGD | 1622.8 | 5 |
| Ng53-75 | RKGPGPGG PGGAGVARGG AGGGP | 1845.0 | 1 |
| SIL-Ng53-75 | RKGPGPGG PGGAGVARGG AGGGP | 1877.1 | 1 |
| Ng52-75 | GRKGPGPGG PGGAGVARGG AGGGP | 1902.1 | 6 |
| Ng51-75 | RGRKGPGPGG PGGAGVARGG AGGGP | 2058.3 | 7 |
| Ng53-78 | RKGPGPGG PGGAGVARGG AGGGPSGD | 2104.3 | 2 |
| Ng50-75 | E RGRKGPGPGG PGGAGVARGG AGGGP | 2187.4 | 8 |
| Ng43-65 | RKKIKSGE RGRKGPGPGG PGGAG | 2205.5 | 9 |
| Ng48-75 | SGE RGRKGPGPGG PGGAGVARGG AGGGP | 2331.5 | 3 |
| Ng42-66 | ARKKIKSGE RGRKGPGPGG PGGAGV | 2375.8 | 10 |
| Ng50-78 | E RGRKGPGPGG PGGAGVARGG AGGGPSGD | 2446.6 | 11 |
| Ng45-75 | KIKSGE RGRKGPGPGG PGGAGVARGG AGGGP | 2701.0 | 12 |
| SIL-Ng45-75 | KIKSGE RGRKGPGPGG PGGAGVARGG AGGGP | 2733.1 | 12 |
| Ng44-75 | KKIKSGE RGRKGPGPGG PGGAGVARGG AGGGP | 2829.2 | 4 |
| Ng43-75 | RKKIKSGE RGRKGPGPGG PGGAGVARGG AGGGP | 2985.4 | 13 |
| Ng43-77 | RKKIKSGE RGRKGPGPGG PGGAGVARGG AGGGPSG | 3129.5 | 14 |
| Ng42-77 | ARKKIKSGE RGRKGPGPGG PGGAGVARGG AGGGPSG | 3200.6 | 15 |
| Ng43-78 | RKKIKSGE RGRKGPGPGG PGGAGVARGG AGGGPSGD | 3244.6 | 5 |
| Ng42-78 | ARKKIKSGE RGRKGPGPGG PGGAGVARGG AGGGPSGD | 3315.7 | 16 |
| Ng40-75 | HMARKKIKSGE RGRKGPGPGG PGGAGVARGG AGGGP | 3324.8 | 17 |
| Ng37-70 | FRGH MARKKIKSGE RGRKGPGPGG PGGAGVARGG | 3345.9 | 18 |
| Ng38-75 | RGH M ARKKIKSGE RGRKGPGPGG PGGAGVARGG AGGGP | 3538.1 | 19 |

**[Table 4]**

| Peptide name | Sequence | Theoretical average m/ z | No. |
|---|---|---|---|
| AcNg1-75ss* | MDCCTENACSKPDDDILDIPLDDPGANAAAAKIQASFRGHMARKKIKSGE RGRKGPGPGGPGGAGVARGGAGGGP | 3700.7 | 28 |
| AcCysNg1-75ss* | MDCCTENACSKPDDDILDIPLDDPGANAAAAKIQASFRGHMARKKIKSGE RGRKGPGPGGPGGAGVARGGAGGGP | 3760.2 | 29 |
| GluNg2-75* | DCCTENACS KPDDDILDIP LDDPGANAAA AKIQASFRGH MARKKIKSGE RGRKGPGPGG PGGAGVARGG AGGGP | 3767.7 | 35 |
| Ng38-78 | RGH MARKKIKSGE RGRKGPGPGG PGGAGVARGG AGGGPSGD | 3797.3 | 20 |
| AcN g1-78ss* | MDCCTENACSKPDDDILDIPLDDPGANAAAAKIQASFRGHMARKKIKSGE RGRKGPGPGGPGGAGVARGGAGGGPSGD | 3830.3 | 30 |
| AcCysNg1-78ss* | MDCCTENACSKPDDDILDIPLDDPGANAAAAKIQASFRGHMARKKIKSGE RGRKGPGPGGPGGAGVARGGAGGGPSGD | 3889.8 | 31 |
| AcGluN g2-78ss* | DCCTENACSKPDDDILDIPLDDPGANAAA AKIQASFRGH MARKKIKSGE RGRKGPGPGG PGGAGVARGG AGGGPSGD | 3917.3 | 32 |
| AcGluNng1-78ss* | MDCCTENACS KPDDDILDIP LDDPGANAAA AKIQASFRGH MARKKIKSGE RGRKGPGPGG PGGAGVARGG AGGGPSGD | 3982.9 | 33 |
| Ng36-78 | SFRGH MARKKIKSGE RGRKGPGPGG PGGAGVARGG AGGGPSGD | 4031.5 | 21 |
| Ng33-75 | IQASFRGH MARKKIKSGE RGRKGPGPGG PGGAGVARGG AGGGP | 4084.7 | 22 |
| Ng33-78 | IQASFRGH MARKKIKSGE RGRKGPGPGG PGGAGVARGG AGGGPSGD | 4343.9 | 23 |
| Ng24-75 | PGANAAAAKIQASFRGH MARKKIKSGE RGRKGPGPGG PGGAGVARGG AGGGP | 4836.5 | 24 |
| SIL-Ng24-75 | PGANAAAAKIQASFRGH MARKKIKSGE RGRKGPGPGG PGGAGVARGG AGGGP | 4868.6 | 24 |
| Recombinant Ng1-78ss* | GSSHHHHHHSSGLVPRGSHM GSMDCCTENACSKPDDDILDIPLDDPGANAAA AKIQASFRGH MARKKIKSGE RGRKGPGPGG PGGAGVARGG AGGGPSGD | 4963.0 | 34 |
| Ng24-78 | PGANAAA AKIQASFRGH M ARKKIKSGE RGRKGPGPGG PGGAGVARGG AGGGPSGD | 5095.7 | 25 |
| Ng7-55 | NACSKPDDDILDIPLDDPGANAAA AKIQASFRGH MARKKIKSGE RGRKGPGPGG PGGAGVARGG AGGGP | 5192.9 | 26 |
| Ng23-78 | DPGANAAA AKIQASFRGH MARKKIKSGE RGRKGPGPGG PGGAGVARGG AGGGP | 5210.8 | 27 |
| AcN g1-75ss | M D C C T E N A C S K P D D D I L D I P L D D P G A N A A A A K I Q A S F R G H M A R K K I K S G E R G R K G P G P G G P G G A G V A R G G A G G G P | 7400.3 | 28 |
| AcCysNg1-75ss | MDCCTENACSKPDDDILDIPLDDPGANAAAAKIQASFRGHMARKKIKSGE RGRKGPGPGGPGGAGVARGGAGGGP | 7519.5 | 29 |
| AcNg1-78ss | MDCCTENACSKPDDDILDIPLDDPGANAAAAKIQASFRGHMARKKIKSGE RGRKGPGPGGPGGAGVARGGAGGGPSGD | 7659.5 | 30 |
| AcCysNg1-78ss | MDCCTENACSKPDDDILDIPLDDPGANAAAAKIQASFRGHMARKKIKSGE RGRKGPGPGGPGGAGVARGGAGGGPSGD | 7778.7 | 31 |
| AcGluNg2-78ss | DCCTENACSKPDDDILDIPLDDPGANAAAAKIQASFRGHMARKKIKSGERGRKGPGPGG PGGAGVARGGAGGGPSGD | 7793.6 | 32 |
| AcGluNg1-78ss | MDCCTENACSKPDDDILDIPLDDPGANAAAAKIQASFRGHMARKKIKSGE RGRKGPGPGGPGGAGVARGGAGGGPSGD | 7964.9 | 33 |
| Recombinant Ng1-78ss | GSSHHHHHHS SGLVPRGSHM GSMDCCTENACSKPDDDILDIPLDDPGANAAA AKIQASFRGH MARKKIKSGE RGRKGPGPGG PGGAGVARGG AGGGPSGD | 9925.0 | 34 |

### [Measured value of Ng peptide]

The peak intensity (NI: normalized intensity) of each Ng peptide normalized with an internal standard peptide (SIL-Ng53-75, SIL-Ng45-75 or SIL-Ng24-75) in each of the four spectra was calculated for each specimen. The value obtained by averaging the four peak intensities was used as the measured value of the Ng peptide of each specimen. Peak intensities (NI) that did not reach the detection limit (S/N < 3) were excluded. The number of data used for averaging the peak intensity (NI) was up to four, but when the number of data was less than three, the peak intensity (NI) was determined to be undetectable and defined as 0.

The reproducibility of the peak intensity (NI) was evaluated with commercially available plasma spiked with Ng peptide and recombinant Ng. As a result, regarding the between-run reproducibility in four days, Ng53-75 had 2.3%CV, Ng48-75 had 7.8%CV, Ng45-75 had 6.9%CV, Ng43-75 had 5.2%CV, Ng24-75 had 3.7%CV, the divalent ion of recombinant Ng had 9.8%CV, and the monovalent ion of recombinant Ng had 15.7%CV. Regarding the within-run reproducibility, four batches were evaluated, and Ng53-75 had 4.9 to 9.7%CV, Ng48-75 had 3.4 to 6.2%CV, Ng45-75 had 2.1 to 5.5%CV, Ng43-75 had 4.1 to 11.2%CV, Ng24-75 had 1.8 to 7.1%CV, the divalent ion of recombinant Ng had 4.6 to 14.5%CV, and the monovalent ion of recombinant Ng had 3.4 to 10.3%CV. This method had an analysis precision of 20%CV or less, and had reproducibility without problems.

Note that, as for the Ng peptide in which both monovalent ions and divalent ions are detected, the same Ng peptide is measured in both cases. Therefore, monovalent ions were analyzed for small Ng peptides having a mass of less than 5000, and divalent ions were analyzed for large Ng peptides having a mass of 5000 or more.

### 2. Measurement of Ng peptide in sample after storage at room temperature

Human plasma (control sample) was thawed, placed at room temperature for about 3 hours, and then re-frozen. Using the human plasma (RT3h-FT1 sample), the measured values of the Ng peptides were obtained in the same manner as described above.

### 3. Variation rate of Ng peptide in plasma

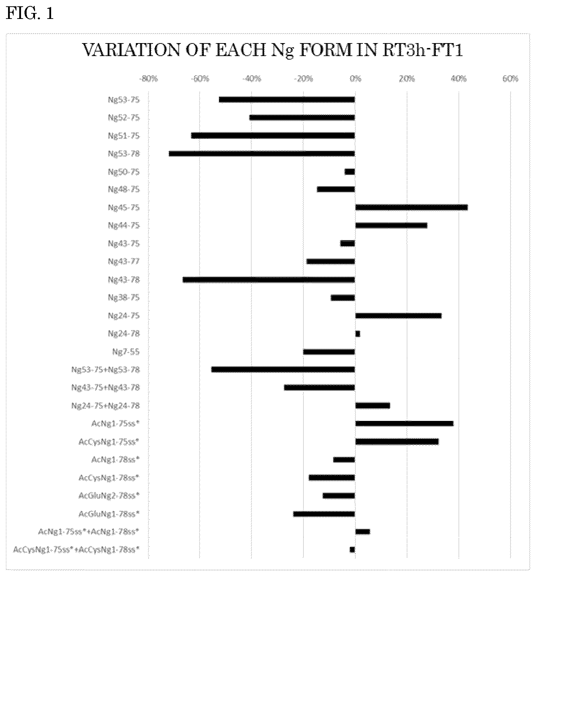

The rate (variation rate) of the measured value of each Ng peptide in the RT3h-FT sample to the measured value of each Ng peptide in the control sample was determined and shown in FIG. 1. FIG. 1 reveals that the amount of the Ng peptide in plasma is increased or decreased by thawing, storage at room temperature, and re-freezing.

### 4. Calculation of ratio of Ng peptide/Ng peptide

Each of the 26 groups of Ng peptides shown in FIG. 1 is divided by each of the 26 groups of Ng peptides to thereby calculate total 650 ratios of Ng peptide/Ng peptide (hereinafter, Ng ratio). Among them, there were 187 (28.8%) Ng ratios with a favorable low variation rate within ±20%, and there were 84 (12.9%) Ng ratios with a low variation rate of 20 to 30% or -20 to -30%. Therefore, it can be seen that these total 271 types of Ng ratios have a suppressed variation rate due to thawing, storage at room temperature, and re-freezing, and can be candidates for an accurate biomarker in consideration of an increase or decrease in Ng peptide in thawing, storage at room temperature, and re-freezing treatment.

### [Verification of Ng ratio as biomarker]

AD_Aβ+ or nonAD_Aβ- is a case which is diagnosed as dementia and in which cognitive function has deteriorated, and thus is a plasma derived from a subject who has already undergone brain neurodegeneration. Therefore, it can be seen that when the Ng ratio in the AD_Aβ+ or nonAD_Aβ- group is increased or decreased in comparison with the CN group, the Ng ratio can be used as a biomarker that varies reflecting brain neurodegeneration. Therefore, the median values of the CN group, the MCI group, the AD_Aβ+ group, and the nonAD_Aβ- group were calculated for 407 types of Ng ratios.

Among 271 types of Ng ratios, those satisfying any of the following screening criteria (1) or (2) were selected, and there were 101 types of Ng ratios. Furthermore, Ng ratios at which the number of specimens was 80% or more (129 specimens or more) in 161 specimens were selected, and there were 89 types of Ng ratios.

### [Screening criteria]

(1) The variation rate is within ±20%, and the median of AD_Aβ+ or nonAD_Aβ-is more than 1.5 times or less than 0.666 times CN.
(2) The variation rate exceeds ±20% but is within ±30%, and the median of AD_Aβ+ or nonAD_Aβ- is more than 2 times or less than 0.5 times CN.

ROC analysis was then performed for these 89 types of Ng ratios in order to evaluate the ability to detect AD_Aβ+ or nonAD_Aβ-. As a result, there were 12 types of Ng ratios showing a very high precision with an AUC of 0.8 or more. Among these Ng ratios, one type of Ng ratio having a reciprocal relationship (relationship in which the numerator and the denominator are merely interchanged) has only a positive/negative correlation with the pathology, and thus was treated as the same biomarker. As a result, 11 types of Ng ratios remained.

For these 16 types of Ng ratios, the median of the CN group, the MCI group, the AD_Aβ+ group, and the nonAD_Aβ- group are shown in Table 5, the difference in the median of the MCI group, the AD_Aβ+ group, and the nonAD _AB- group from the CN group is shown in Table 6, the test of the difference in the median is shown in Table 7, and the AUC is shown in Table 8.

**[Table 5]**

| | | Number of detections | Median of each group | | | |
|---|---|---|---|---|---|---|
| | | | CN | MCI | AD | nonAD |
| (1) | Ng53-75/Ng52-75 | 160 | 2.835 | 3.572 | 6.496 | 5.026 |
| (2) | Ng53-78/Ng51-75 | 135 | 0.831 | 1.168 | 2.713 | 2.386 |
| (3) | Ng43-77/Ng43-75 | 130 | 0.019 | 0.021 | 0.030 | 0.029 |
| (4) | Ng43-77/NgAc1-78ss* | 130 | 0.062 | 0.087 | 0.156 | 0.131 |
| (5) | Ng43-77/[AcNg1-75ss*+AcNg1-78ss*] | 130 | 0.032 | 0.050 | 0.105 | 0.091 |
| (6) | Ng43-77/[AcCysNg1-75ss*+AcCysNg1-78ss*] | 130 | 0.013 | 0.018 | 0.042 | 0.036 |
| (7) | Ng43-77/[Ng24-75+Ng24-78] | 130 | 0.189 | 0.216 | 0.625 | 0.550 |
| (8) | Ng43-78/Ng51-75 | 134 | 5.098 | 8.769 | 17.608 | 19.489 |
| (9) | Ng24-78/AcCysNg1-75ss* | 159 | 0.076 | 0.081 | 0.131 | 0.121 |
| (10) | [Ng53-75+Ng53-78]/Ng52-75 | 160 | 3.244 | 3.894 | 7.242 | 5.772 |
| (11) | [Ng53-75+Ng53-78]/Ng51-75 | 135 | 9.603 | 13.446 | 24.971 | 24.616 |

**[Table 6]**

| | Number of detections | Difference in median of each group | | |
|---|---|---|---|---|
| | | CN vsMCI | CN vs AD_Aβ + | CN vs nonAD_Aβ - |
| Ng53-75/Ng52-75 | 160 | 1.260 | 2.291 | 1.773 |
| Ng53-78/Ng51-75 | 135 | 1.405 | 3.265 | 2.872 |
| Ng43-77/Ng43-75 | 130 | 1.099 | 1.585 | 1.499 |
| Ng43-77/NgAc1-78ss* | 130 | 1.406 | 2.523 | 2.125 |
| Ng43-77/[AcNg1-75ss*+AcNg1-78ss*] | 130 | 1.534 | 3.242 | 2.821 |
| Ng43-77/[AcCysNg1-75ss*+AcCysNg1-78ss*1 | 130 | 1.331 | 3.175 | 2.765 |
| Ng43-77/[Ng24-75+Ng24-78] | 130 | 1.141 | 3.303 | 2.905 |
| Ng43-78/Ng51-75 | 134 | 1.720 | 3.454 | 3.823 |
| Ng24-78/AcCysNg1-75ss* | 159 | 1.071 | 1.729 | 1.605 |
| [Ng53-75+Ng53-78]/Ng52-75 | 160 | 1.200 | 2.232 | 1.779 |
| [Ng53-75+Ng53-78]/Ng51-75 | 135 | 1.400 | 2.600 | 2.563 |

**[Table 7]**

| | Test of difference in median for each group of 16 Ng peptides meeting marker screening criteria (p-value, significance level p < 0.05) | | | |
|---|---|---|---|---|
| | Kruskal-Wallis test (one-way ANOVA) | Wilcoxon rank-sum test (Holm correction) | | |
| | | CN vs MCI | CN vs AD_Aβ + | CN vs nonAD_Aβ - |
| Ng53-75/Ng52-75 | <0.0001 | 0.3785 | <0.0001 | <0.0001 |
| Ng53-78/Ng51-75 | <0.0001 | 0.1497 | <0.0001 | <0.0001 |
| Ng43-77/Ng43-75 | <0.0001 | 0.2104 | <0.0001 | <0.0001 |
| Ng43-77/AcNg1-78ss* | <0.0001 | 0.2053 | <0.0001 | <0.0001 |
| Ng43-77/[AcNg1-75ss*+AcNg1-78ss*] | <0.0001 | 0.2264 | <0.0001 | <0.0001 |
| Ng43-77/[AcCysNg1-75ss*+AcCysNg1-78ss*] | <0.0001 | 0.3332 | <0.0001 | <0.0001 |
| Ng43-77/[Ng24-75+Ng24-78] | <0.0001 | 0.3869 | <0.0001 | <0.0001 |
| Ng43-78/Ng51-75 | <0.0001 | 0.1497 | <0.0001 | <0.0001 |
| Ng24-78/AcCysNg1-75ss* | <0.0001 | 0.5997 | <0.0001 | <0.0001 |
| [Ng53-75+Ng53-78]/Ng52-75 | <0.0001 | 0.4347 | <0.0001 | <0.0001 |
| [Ng53-75+Ng53-78]/Ng51-75 | <0.0001 | 0.0282 | <0.0001 | <0.0001 |

**[Table 8]**

| | AUC in ROC analysis | | |
|---|---|---|---|
| | CN vs MCI | CN vs AD_Aβ + | CN vs nonAD_Aβ - |
| Ng53-75/Ng52-75 | 0.579 | 0.884 | 0.801 |
| Ng53-78/Ng51-75 | 0.637 | 0.933 | 0.898 |
| Ng43-77/Ng43-75 | 0.610 | 0.897 | 0.839 |
| Ng43-77/NgAc1-78ss* | 0.610 | 0.804 | 0.770 |
| Ng43-77/[AcNg1-75ss*+AcNg1-78ss*] | 0.605 | 0.829 | 0.796 |
| Ng43-77/[AcCysNg1-75ss*+AcCysNg1-78ss*] | 0.585 | 0.829 | 0.793 |
| Ng43-77/[Ng24-75+Ng24-78] | 0.579 | 0.861 | 0.786 |
| Ng43-78/Ng51-75 | 0.637 | 0.854 | 0.876 |
| Ng24-78/AcCysNg1-75ss* | 0.547 | 0.813 | 0.765 |
| [Ng53-75+Ng53-78]/Ng52-75 | 0.569 | 0.890 | 0.814 |
| [Ng53-75+Ng53-78]/Ng51-75 | 0.707 | 0.937 | 0.896 |

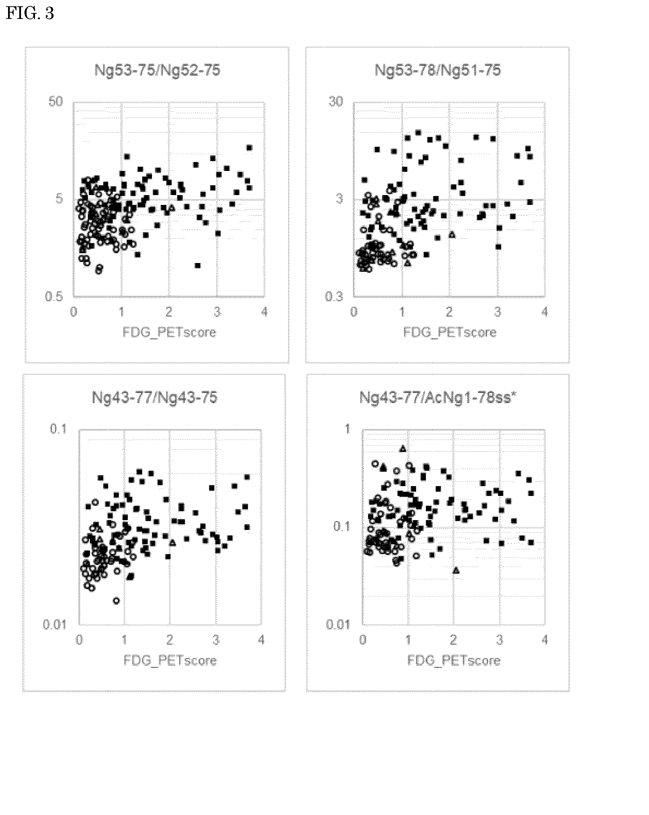

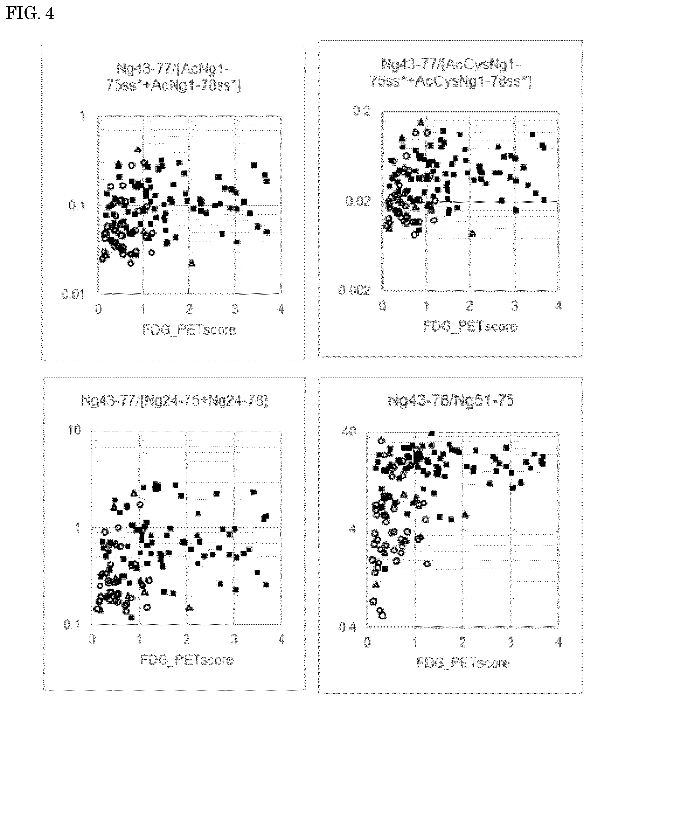

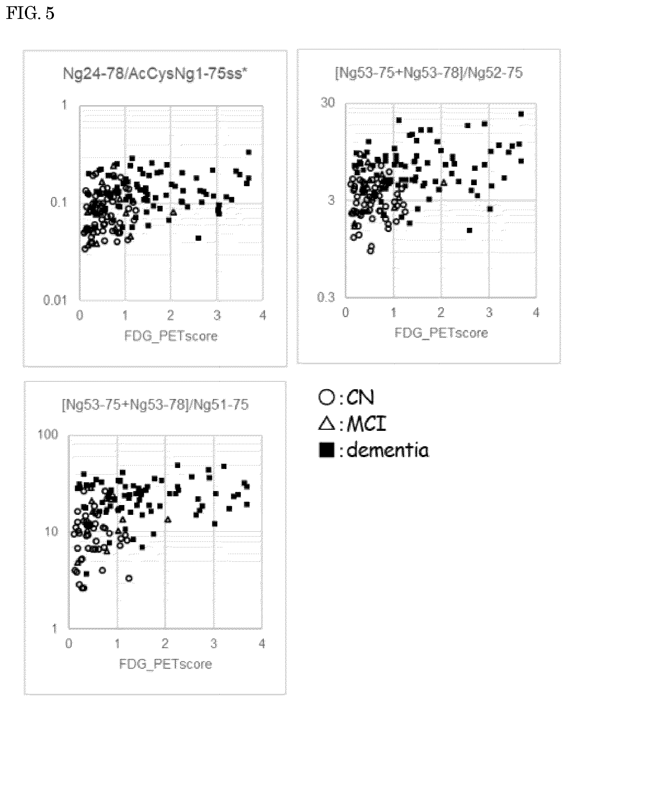

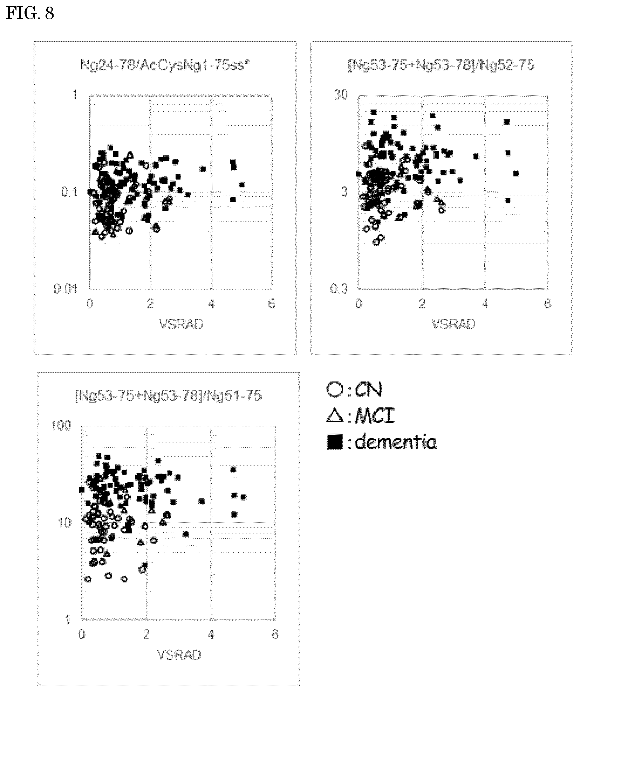

The relationship between 11 types of Ng ratio biomarkers and FDG-PET, VSRAD, and MMSE is shown in FIGs. 3 to 8. MMSE is an examination score for evaluating cognitive function. FDG-PET is a score indicating degradation of function of brain nerve cells and synapses by evaluating cerebral glucose metabolism. VSRAD is a score indicating brain atrophy caused by loss of nerve cells in the medial temporal lobe by MRI examination. These examination scores are used as indices of neurodegeneration in the brain. A higher score of the FDG-PET and VSRAD indicates advance of neurodegeneration, and a lower score of MMSE indicates advance of neurodegeneration.

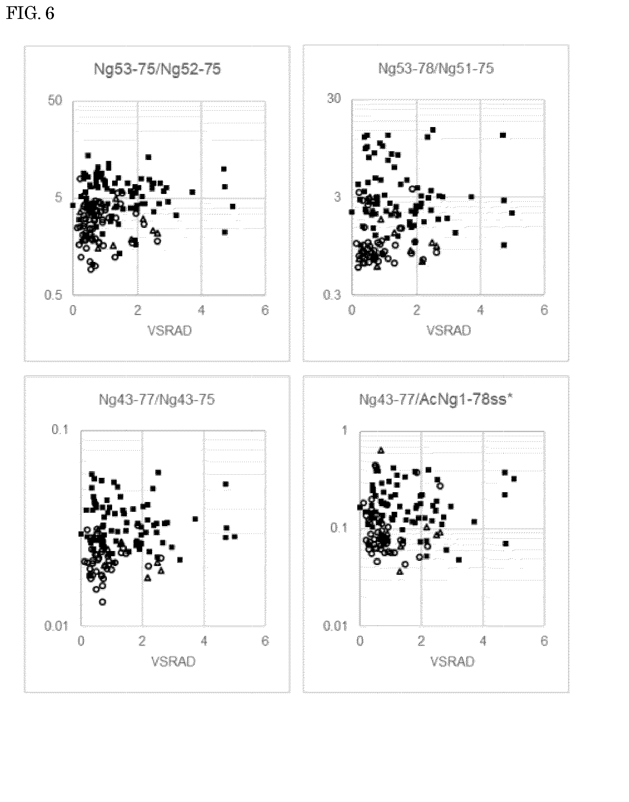

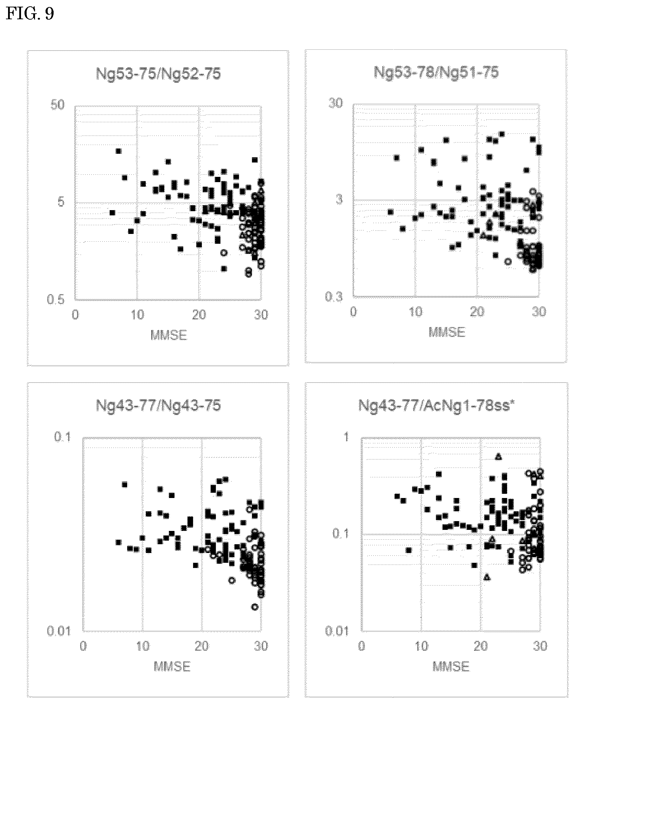

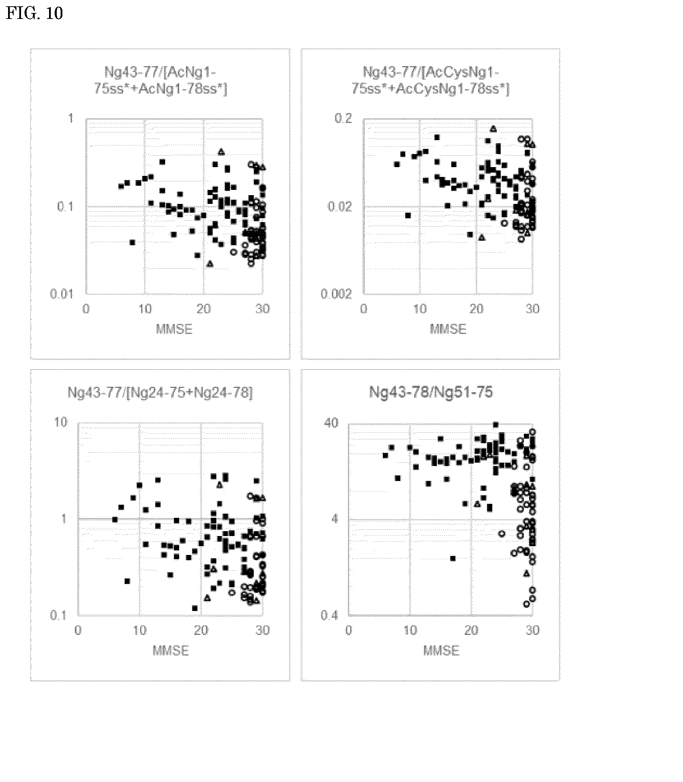

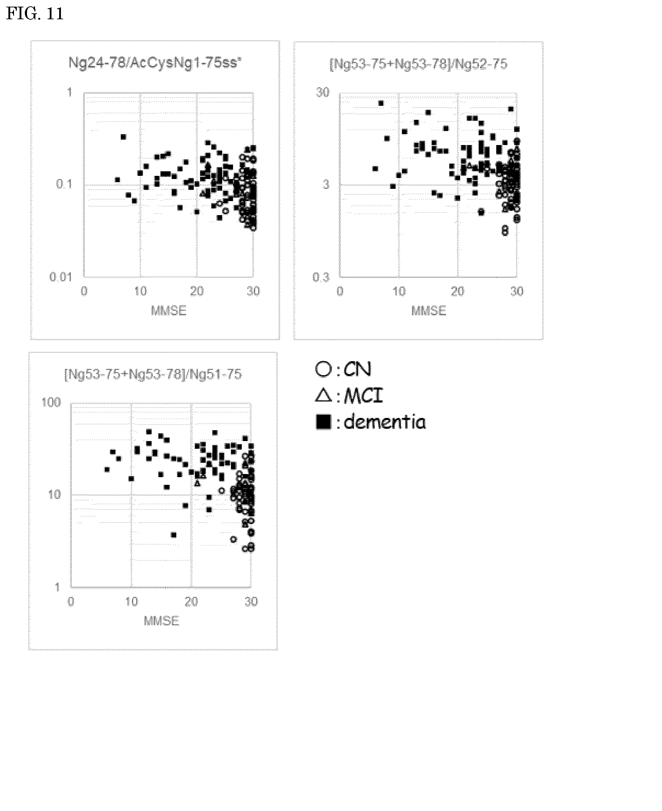

In the relationship between each of Ng ratio biomarkers and FDG-PET, VSRAD, and MMSE, in a case where FDG-PET or VSRAD shows a high value, the Ng ratio also tends to be relatively high (FIGs. 3 to 5, FIGs. 9 to 11). Similarly, even in a case where MMSE shows a low value, the Ng peptide ratio tends to be relatively high (FIGs. 6 to 8). These results indicate that these Ng ratios capture the variations of brain neurodegeneration that can be detected with FDG-PET, VSRAD, and MMSE. Also, the Ng ratio tends to be high in Dementia cases that cannot be supplemented by FDG-PET, VSRAD, and MMSE. This indicates that brain neurodegeneration of Dementia cases that are overlooked by FDG-PET, VSRAD, and MMSE is also captured.

The difference between the CN group and the AD_Aβ+ or nonAD _AB- group may be a difference caused by the difference between the AB positive and AB negative as AD pathology. However, there was no statistically significant difference in the difference in the median value between the AB positive group and the AB negative group in the CN group and the difference in the median value between the AB positive group and the AB negative group in the case group developing dementia, that is, between the AD_Aβ+ group and the nonAD_Aβ- group (Table 9).

**[Table 9]**

| Test of difference in median between A *β* positive and A *β* negative (p-value, significance level p < 0.05) | | |
|---|---|---|
| | Wilcoxon rank-sum test (Holm correction) | |
| | CN_Aβ - vs CN_Aβ + | nonAD_Aβ - vs AD_Aβ + |
| Ng53-75/Ng52-75 | 0.7714 | 0.2471 |
| Ng53-78/Ng51-75 | 0.6306 | 0.3927 |
| Ng43-77/Ng43-75 | 0.1742 | 0.2107 |
| Ng43-77/NgAc1-78ss* | 0.6818 | 0.4469 |
| Ng43-77/[AcNg1-75ss*+AcNg1-78ss*] | 0.3233 | 0.6428 |
| Ng43-77/[AcCysNg1-75ss*+AcCysNg1-78ss*] | 0.3499 | 0.5533 |
| Ng43-77/[Ng24-75+Ng24-78] | 0.5249 | 0.4077 |
| Ng43-78/Ng51-75 | 0.6339 | 0.4665 |
| Ng24-78/AcCysNg1-75ss* | 0.8356 | 0.7727 |
| [Ng53-75+Ng53-78]/Ng52-75 | 0.8395 | 0.2117 |
| [Ng53-75+Ng53-78]/Ng51-75 | 0.7397 | 0.5336 |

The above results reveal that 11 types of Ng peptide ratio biomarkers do not vary reflecting only AD pathology, but also dementia due to brain neurodegeneration (including synaptic disorder).

### <Experimental Example 2>

### [Multivariate analysis of Ng ratio biomarkers]

The predictive probability of dementia by logistic regression analysis was calculated using 11 types of Ng ratio biomarkers, and ROC analysis was performed. As a result, a higher AUC was obtained in CN vs AD_Aβ+ and CN vs nonAD_Aβ- in each of 11 types of Ng peptide biomarkers (Table 10).

Normalization was performed using the average and standard deviation of Ng53-78/Ng51-75 and [Ng53-75 + Ng53-78]/Ng51-75 of 161 specimens, respectively, to calculate a z-score. Then, the z-scores of Ng53-78/Ng51-75 and [Ng53-75 + Ng53-78]/Ng51-75 were averaged to calculate a synthetic z-score (Table 11). As a result, a higher value of AUC than any of 11 types of Ng ratio biomarkers was obtained in CN vs AD_Aβ+, CN vs nonAD_Aβ-, and CN vs MCI (Table 11).

The above results indicate that the ability to detect brain neurodegeneration is improved by multivariate analysis.

**[Table 10]**

| Method | | Median of each group of variables calculated by multivariate analysis | | | |
|---|---|---|---|---|---|
| | | CN | MCl | AD_Aβ + | nonAD_Aβ - |
| Logistic regression | 11Ng Ratio | 0.074 | 0.088 | 1.000 | 0.999 |
| Synthetic z-score | Ng53-78/ Ng 51-75 and [Ng 53-75+ Ng 53-78]/ Ng 51-75 | -0.779 | -0.563 | 0.379 | 0.343 |

**[Table 11]**

| Method | | AUC in ROC analysis using variables calculated by multivariate analysis | | |
|---|---|---|---|---|
| | | CN vs MCI | CN vs AD_Aβ + | CN vs nonAD_Aβ - |
| Logistic regression | 11 Ng Ratio | 0.496 | 0.982 | 0.962 |
| Synthetic z-score | Ng53-78/ Ng 51-75 and [Ng 53-75+ Ng 53-78]/ Ng 51-75 | 0.702 | 0.945 | 0.903 |

### <Comparison with markers of Non-Patent Documents 5 to 7>

Non-Patent Documents 5 to 7 disclose Ng molecules shown in Table 12 as markers. In these documents, comparison between two groups of control (healthy subject) and AD is performed, and the p-value is calculated. This is shown in Table 12. Typically, a larger difference between the two groups indicates a lower p-value. The lowest p-value among the Ng molecules shown in Table 12 was 0.002 of Ng48-76. On the other hand, the difference between the groups of NC vs AD_Aβ+ of 11 types of Ng markers, which corresponds to the difference between the two groups of control (healthy subject) and AD, indicates p < 0.0001 (Table 7). It can be said that the difference between the two groups in the Ng marker is larger than that of the Ng molecule shown in Table 12.

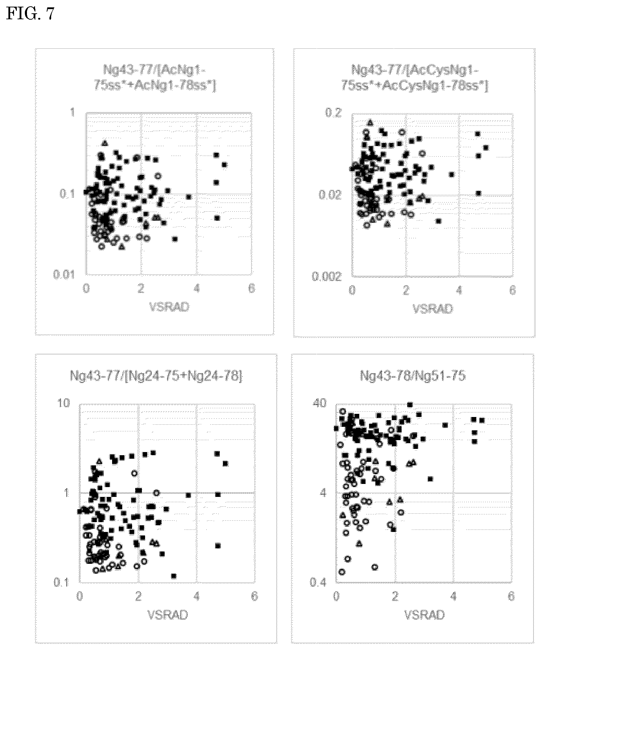

As a test method, a Mann-Whitney U test was used in Non-Patent Document 5, a Mann-Whitney U test was used in Non-Patent Document 6, and a Wilcoxon rank-sum test was used for the 11 types of Ng markers. Since the Mann-Whitney U test and the Wilcoxon rank-sum test are equivalent, these test methods are used for the markers of the Non-Patent Documents and the 11 types of markers. In Table 12, the expression "total FL" is the sum of three peaks of Ng1-78 slightly different in post-translational modification illustrated in FIG. 7c of Non-Patent Document 5.

**[Table 12]**

| Document | Sample | Molecule | Control (healthy subject) vs AD P-value |
|---|---|---|---|
| Non-Patent Document 5 | Brain tissue | Ng53-75/total FL ratio | 0.035 |
| | | Ng53-78/total FL ratio | 0.023 |
| | | Ng51-78/total FL ratio | 0.046 |
| | | Ng48-76/total FL ratio | 0.019 |
| | | Ng48-78/total FL ratio | 0.019 |
| | | Ng44-76/total FL ratio | 0.029 |
| | | Ng42-78/total FL ratio | 0.016 |
| | | Ng41-78/total FL ratio | 0.027 |
| Non-Patent Documents 6 and 7 | CSF | Ng48-76 | Study1, 0.002 Study 2, between 0.05 and 0.01 |

The Ng molecules shown in Table 12 are supposed to be detected in the detection of the Ng molecules in the blood sample shown in Experimental Example 1 if these Ng molecules were present in the sample at a concentration equal to or higher than the detection limit in this measurement method. However, among these Ng molecules, Ng molecules other than Ng53-75 and Ng53-78 were not detected (see Tables 3 and 4). Therefore, it can be said that molecular markers other than Ng53-75 and Ng53-78 disclosed in Non-Patent Documents 5 to 7 are not effective as markers in the blood sample.

## Claims

1. A method for estimating a brain neurodegenerative condition, the method comprising:
a measurement step of measuring a blood sample collected from a subject to obtain measured values of a plurality of neurogranin-related peptides; and
a use step of calculating at least one of the values of the ratios (1) to (11) using the measured values of the plurality of neurogranin-related peptides, and using at least one of the values of the ratios to determine a neurodegenerative condition:
(1) a ratio of a measured value of Ng53-75 to a measured value of Ng52-75;
(2) a ratio of a measured value of Ng53-78 to a measured value of Ng51-75;
(3) a ratio of a measured value of Ng43-77 to a measured value of Ng43-75;
(4) a ratio of a measured value of Ng43-77 to a measured value of AcNg1-78ss;
(5) a ratio of a measured value of Ng43-77 to a sum of measured values of AcNg1-75ss and AcNg1-78ss;
(6) a ratio of a measured value of Ng43-77 to a sum of measured values of AcCysNg1-75ss and AcCysNg1-78ss;
(7) a ratio of a measured value of Ng43-77 to a sum of measured values of Ng24-75 and Ng24-78;
(8) a ratio of a measured value of Ng43-78 to a measured value of Ng51-75;
(9) a ratio of a measured value of Ng24-78 to a measured value of AcCysNg1-75ss;
(10) a ratio of a sum of measured values of Ng53-75 and Ng53-78 to a measured value of Ng52-75; and
(11) a ratio of a sum of measured values of Ng53-75 and Ng53-78 to a measured value of Ng51-75.

2. The estimation method according to claim 1, wherein, in the use step, when the value of the ratio is higher than a predetermined value, it is determined that neurodegeneration has occurred in the brain of the subject.

3. The estimation method according to claim 1, wherein in the use step, the value of the ratio is compared with a preset reference value of normal cognitive function.

4. The estimation method according to claim 1, wherein
the measurement step is performed a plurality of times over time, and
the use step is a step of comparing a plurality of values of ratios obtained and calculated by performing the step a plurality of times.

5. The estimation method according to claim 1, wherein
in the use step, two values of ratios are selected from among ratios obtained by using the measured values of the plurality of neurogranin-related peptides,
a variable is calculated using multivariate analysis, and
the variable is used to determine a neurodegenerative condition.

6. The method for estimating a brain neurodegenerative condition according to claim 1, wherein, in the use step, at least one of the values of the ratios (1) to (2) is used.

7. The method for estimating a brain neurodegenerative condition according to claim 1, wherein, in the use step, at least one of the values of the ratios (3) to (7) is used.

8. The method for estimating a brain neurodegenerative condition according to claim 1, wherein in the use step, at least one of the values of the ratios (10) to (11) is used.

9. The estimation method according to claim 1, wherein in the measurement step, mass spectrometry is performed on the blood sample to obtain measured values of peak intensities for the plurality of neurogranin-related peptides.

10. The estimation method according to claim 9, wherein in the measurement step, affinity purification is performed on the blood sample before mass spectrometry.
